# EUROPEAN PATENT APPLICATION

(11) **EP 2 068 142 A1**
(43) Date of publication of application: **10.06.2009**
(21) Application number: 07023483.6
(22) Date of filing: 04.12.2007
(51) Int. Cl.: G01N 27/12

(54) **Use of a nanoparticle film having metal ions incorporated**

(71) Applicant: Sony Corporation, Minato-ku Tokyo 108-0075 (JP)
(72) Inventor: Joseph, Yvonne, 70327 Stuttgart (DE); Krasteva, Nadejda, 70327 Stuttgart (DE)
(74) Representative: Appelt, Christian W.

(57) **Abstract**

The present invention relates to the use of a nanoparticle film having metal ions incorporated and to a method of detecting a gaseous or volatile or liquid analyte in a medium.

## Description

The present invention relates to the use of a nanoparticle film having metal ions incorporated and to a method of detecting a gaseous or volatile or liquid analyte in a medium.

Nanoparticle films are useful in many applications, such as molecular electronic devices, for example chemical sensors. In order to be stable, nanoparticles in such films can be capped or interlinked by capping molecules or linker molecules. One method of forming such nanoparticle films which are interlinked with molecules is the layer-by-layer self-assembly (EP 1 022 560). Here, substrates are alternately immersed into nanoparticle solutions/dispersions and solutions of organic molecules, such as dithiols (Joseph, et al., J. Phys. Chem. B 2003, 107,7406) and bis(dithiocarbamates) (Wessels et al., J. An. Chem. Soc., 2004, 126, 3349). This results in an assembly of the material in a nanoparticle film wherein the nanoparticles are interlinked. Whilst the nanoparticles in the film are most important for the conductivity and the high surface to volume ratio of the materials, the organic molecules functioning as linkers or capping molecules determine the physical and chemical properties of the materials (EP 1 215 485). Therefore, a broad variety of materials with tuned properties can be achieved by choosing appropriate linker molecules. Commonly, these organic linker molecules have to be synthesized and purified before hand.

The layer-by-layer self-assembly process referred to above has many advantages. The main advantage is the reproducibility of the preparation and the structural control of the film.

There is an ongoing interest in the development of sensors and sensor devices ("electronic noses") that act as analogues of the human or animal olfactory system. There are a number of analytes that the human or animal alfactory system appears to be particularly sensitive to which are volatile sulfur compounds, in particular thiols, and biogenic amines. Artificial sensors have, however, so far failed in being sensitive enough to mimic the natural human or animal olfactory system and to be able to detect these compounds with enhanced sensitivity.

Accordingly, it was an object of the present invention to provide for an enhanced sensitivity in artificial sensors, with respect to analytes. Furthermore, it was also an object of the present invention to increase the sensitivity of artificial sensors, in particular with respect to volatile sulfur compounds and biogenic amines.

All these objects are solved by the use of a nanoparticle film, said nanoparticle film having metal ions incorporated, for detecting the presence or absence of a gaseous or volatile or liquid analyte.

Preferably, in said nanoparticle film, nanoparticles are linked by organic linkers.

In one embodiment said gaseous or volatile or liquid analyte is a metabolite of an organism.

In one embodiment said gaseous or volatile or liquid analyte is a nitrogen containing compound or a sulphur containing compound.

In one embodiment said gaseous or volatile or liquid analyte is selected from the group comprising volatile sulphur compounds, such as methylmercaptane, hydrogensulfide, dimethylsulfide, biological amines, such as cadaverine, 1-butylamine, methylamine, isobutylamine, putrescine, histamine, tyramine, indole, skatole and phenethylamine.

In one embodiment said metal ions are selected from the group comprising Mg²⁺, Ca²⁺, Pb^{2+/4+}, Mn^{2+/3+/4+/6+/7+}, Co^{2+/3+}, Fe^{2+/3+}, Cu^{+/2+}, Ag⁺, Zn²⁺, Cd²⁺, Hg^{+/2+}, Cr^{2+/3+/6+}, Ce^{3+/4+}, Pd^{2+/4+}, Pt^{2+/4+6+} , Cu^{+/2+}, Fe²⁺/³⁺, Sn⁴⁺ , Au^{+/2+/3+/5+}, Ni ²⁺, Rh^{+/2+/3+/4+}, Ru^{2+/3+/4+/6+/8+}, Mo^{2+/3+/4+/5+/6+}

In one embodiment said metal ions have been incorporated in said nanoparticle film by exposing said nanoparticle film to a solution of metal ions, said film, prior to said incorporation of metal ions, being devoid of metal ions.

In one embodiment said metal ions incorporated in said film are not complexed in coordination complexes before incorporation and are not coordinated by carboxylate groups of mercaptoundecanoic acid.

In one embodiment, said metal ions, upon incorporation into said film, react with said film and become oxidised or reduced, preferably to a metallic state.

In one embodiment said gaseous or volatile or liquid analyte is present in a medium to which said nanoparticle film is exposed, and said analyte is present in said medium at a concentration in the range of from 10 parts per trillion to 100 parts per million.

In one embodiment said analyte is an amine and said metal ions are Cu²⁺.

In another embodiment said analyte is methylmercaptane and said metal ions are Ag⁺ or Hg²⁺ or combinations thereof.

In one embodiment said organic linkers are bi- or polyfunctional linkers, preferably selected from the group comprising thiols such as C₅-C₃₀-alkane dithiols, such as 1,12-dodecanedithiol, or amines or dithiocarbamates such as 1,4,10,13-tetraoxa-7,16-bisdithiocarbamate-cyclo-octadecane of thiocticacids or isocyanates.

The objects of the present invention are also solved by a method of detecting a gaseous or volatile or liquid analyte in a medium, said method comprising:
providing a sensor comprising a nanoparticle film, in which film, preferably, said nanoparticles are linked by organic linkers, said film having metal ions incorporated, said nanoparticle film showing a change in electrical conductivity in the presence of a gaseous or volatile or liquid analyte,
exposing said sensor to said medium, and measuring a response based on said change of conductivity if said analyte is present, said film, said metal ions and said analyte being as defined above.

As used herein, the term "organic linker" is meant to refer to an organic molecule in which there are at least two independent sides that enable the binding of said linker molecule to nanoparticles and/or to the substrate. Preferably, such linker is "polyfunctional", which means that it has more than one side that enables such binding. "Functionality" in this context, therefore refers to the capability of binding to nanoparticles.

The term "nanoparticle", as used herein, is meant to refer to particles the average dimensions of which are < 1 µm, preferably ≤ 500 nm, more preferably ≤ 300 nm, and most preferably ≤ 100 nm.

Preferably, the metal ions to be incorporated by the method according to the present invention are selected from the main group metals, the transition metals and/or the rare earth metals. Particular examples are Mg²⁺, Ca²⁺, Pb^{2+/4+} Mn^{2+/3+/4+/6+n+7+}, Co^{2+/3}, Fe^{2+/3+}, Cu^{+/2+}, Ag⁺, Zn²⁺, Cd²⁺, Hg^{+/2+}, Cr^{2+/3+/6+}, Ce^{3+/4+}, Pd^{2+/4+}, Pt^{2+/4+6+}, Cu^{+/2+}, Fe²⁺/³⁺, Sn⁴⁺, Au^{+/2+/3+/5+}, Ni ²⁺, Rh^{+/2+/3+/4+}, Ru^{2+/3+/4+/6+/8+}, Mo^{2+/3+/4+/5+/6+}
During the process of incorporation the metal ions may partly or completely react with the nanoparticular film and thus become themselves oxidized or reduced (up to metallic state).

The term "volatile sulfur compounds" as used herein is meant to refer to gaseous compounds or vaporized compounds containing sulfur, in particular those of a biogenic origin. Typical examples of such volatile sulfur compounds are hydrogen sulfide and methylmercaptane. For a review of "volatile sulfur compounds" and their roles as biomarkers, see also Richter et al., Arch. Oral Biol., 9:47-53 (1964) and Tonzetich, J. Int. Dent. J. 28:309-319 (1978).

The term "biogenic amines" is used herein synonymously with the term "biological amines" and refers to amines which are present in an organism or are generated by its own metabolism or the activity of bacteria. Typical examples of such biological amines are cadaverine, 1-butylamine, methylamine, isobutylamine, putrescine, histamine, thyramine, indole, skatole and phenethylamine.

The term "coordination complex", as used herein is meant to refer to a complex of a metal which is acting as a central atom and is surrounded by a number of ligands

The term "gaseous or volatile or liquid" as used herein is also meant to include those compounds which, at room temperature, are liquid but which have a high vapor pressure and therefore are likely to develop vapors. Likewise the analyte that may be detected by the present invention may also be in the form of a liquid. The inventors have surprisingly found that by incorporating metal ions into a nanoparticle film having organic linker molecules, the sensitivity of such a sensor can be dramatically enhanced. For example by incorporating metal ions into a nanoparticle film having organic linkers, it is possible to reach sensitivity ranges below 1ppb.

Consequently, by using a simple measure, namely the incorporation of metal ions into nanoparticle films, it is possible to drastically increase the sensitivity of nanoparticle film-based sensors.

There are different ways of incorporating metals or metal ions into nanoparticle film. One method is the simple exposure of a nanoparticle film to a solution of metal ions, which method is disclosed in co-pending patent application "A method of preparing a nanoparticle film having metal ions incorporated", applicant's reference no. FB19530 filed on the same day as the present application. The content of this co-pending patent application is incorporated herein in its entirety by reference thereto.

According to the present invention, nanoparticle films having metal ions incorporated have a considerably higher sensitivity to analytes in comparison with nanoparticle films without such metals, i.e. "undoped" nanoparticle films. Moreover, in accordance with the present invention, the sensitivity of the nanoparticle film can be tuned and geared towards different analytes, simply by varying and choosing the appropriate metal ion. Furthermore, sensor arrays may be formed, in which different regions of a nanoparticle film with organic linkers and having metal ions incorporated, have different metal ions, such that one region has a first type of metal ion incorporated and, accordingly, a higher sensitivity to one type of analyte, whereas a second region has another type of metal ion incorporated and, consequently a higher sensitivity to another type of analyte and so forth Each of these different regions may be viewed as a separate sensor which needs to be addressed separately. In such a sensor array, one sensor comprising a given nanoparticle film and having one type of metal or metal ions incorporated, has a higher sensitivity to one type of analyte, while another sensor within the array comprising compositionally the same nanoparticle film but having a second type of metal or metal ions incorporated, has a higher sensitivity to a second type of analyte, and so forth.

Moreover, reference is made to the figures wherein
figure 1 shows response traces of a AuDAC-film having Co-ions incorporated towards 100 ppb 1-butylamin in comparison with "undoped" AuDAC, i.e. without such Co-ions,
figure 2 shows response traces of AuDT having Cu-ions incorporated towards 10 ppb cadaverine in comparison with "undoped" AuDT,
figure 3 shows response traces of AuDT having Hg-ions incorporated towards 20 ppb methylmercaptane in comparison with "undoped" AuDT,
figure 5 shows response traces of AuDAC having various metal ions incorporated, towards 3030 ppm 1-butylamin in comparison with "undoped" AuDAC,

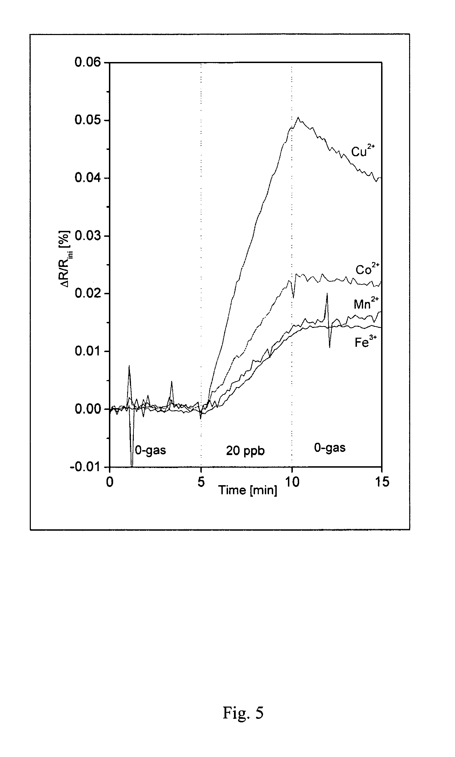

figure 5 shows response traces of AuDT having different metal ions incorporated towards 10 ppb cadaverine, and

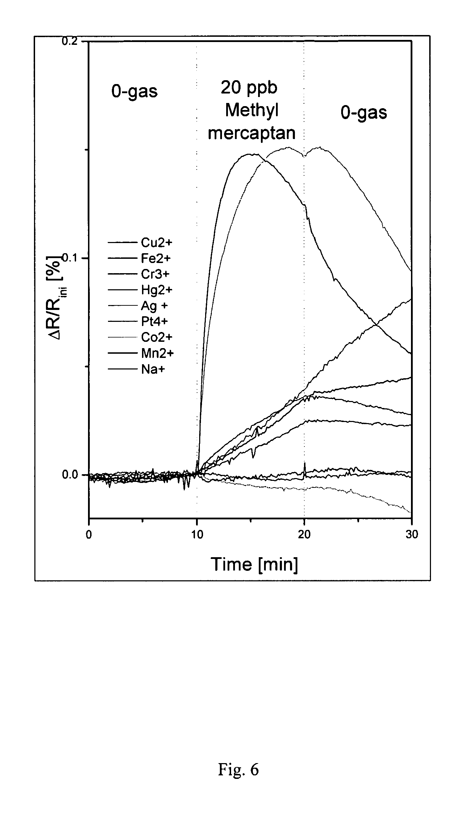

figure 6 shows response traces of AuDT having different metal ions incorporated towards 20 ppb methyl mercaptane.

Moreover, reference is made to the following examples which are given to illustrate, not to limit the present invention.

### Example 1:

### Enhanced sensitivity for 100 ppb 1-butylamine detection by incorporation of Co ions in AuDAC

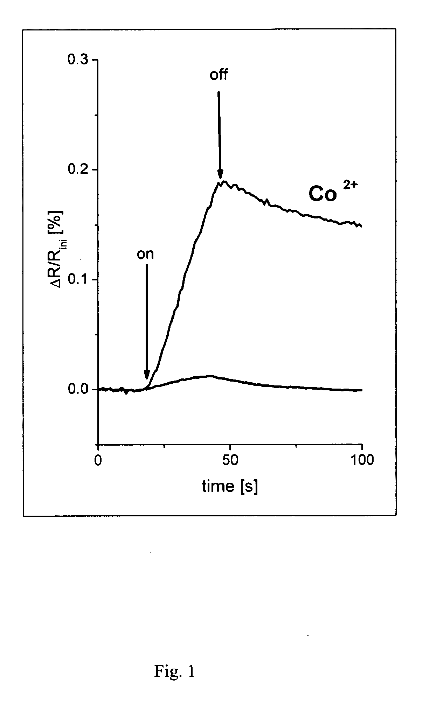

A nanoparticular film was prepared by layer-by layer growth as described in EP 1022560. Gold nanoparticles were used as metal component whereas 1,4,10,13-tetraoxa-7,16-diaza-cyclooctadecane was reacted in-situ with CS₂ and Triethylamine in Toluene to the respective bisdithiocarbamate (DAC) as described in EP06006881 which was used as linker solution. Afterwards the prepared Films were immersed in Co(NO₃)₂*6H₂O solved in isopropanol. Figure 1 shows the responses towards 100 ppb 1-butylamine of the film compared with a reference sensor immersed only in pure isopropanol. The response of the metal doped film is much higher than those of the undoped material.

### Example 2:

### Enhanced sensitivity for 10 ppb cadaverine detection by incorporation of Cu ions in AuDT

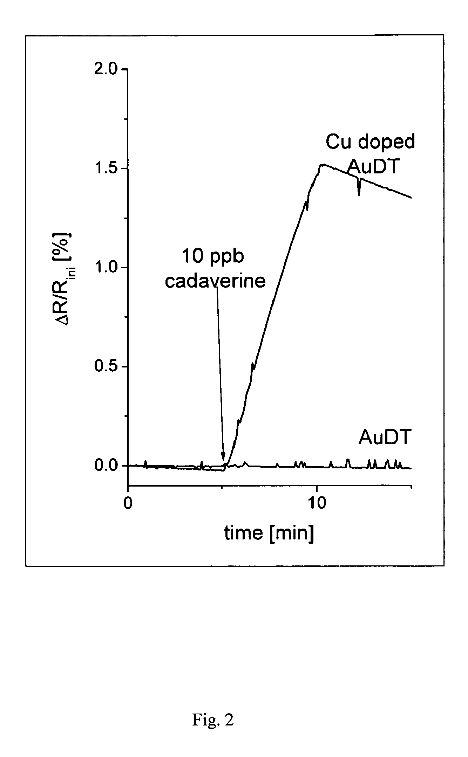

A nanoparticular film was prepared by layer-by layer growth as described in EP1022560. Gold nanoparticles were used as metal component whereas 1,12-Dodecanedithiol was used as linker in toluene solution. Afterwards the prepared films were immersed in Cu(ClO₄)₂*6H₂O solved in isopropanol. Figure 2 shows the responses towards 10 ppb cadaverine of the film compared with a reference sensor immersed only in pure isopropanol. The response of the metal doped film is much higher than those of the undoped material.

### Example 3:

### Enhanced sensitivity for 20 ppb methylmercaptane detection by incorporation of Hg ions

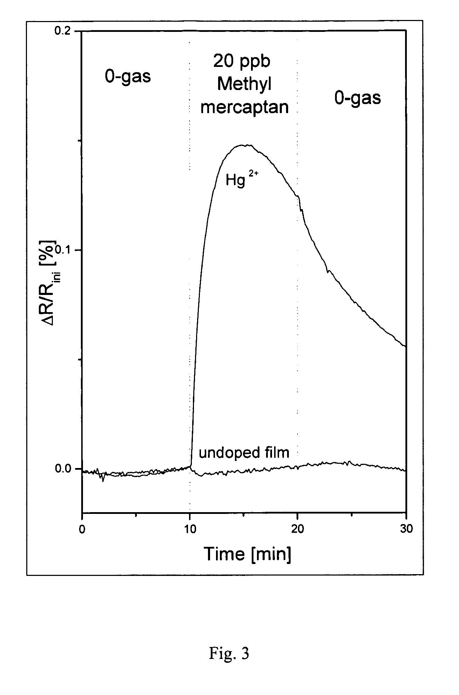

A nanoparticular film was prepared by layer-by layer growth as described in EP1022560. Gold nanoparticles were used as metal component whereas 1,12-Dodecanedithiol was used as linker in toluene solution. Afterwards the prepared Films were immersed in Hg(ClO₄)₂ solved in methanol. Figure 3 shows the responses towards 20 ppb Methylmercaptane of the film compared with a reference sensor immersed only in pure methanol. The response of the metal doped film is much higher than those of the undoped material.

### Example 4:

### 1-Butylamine detection with AuDAC doped with different metals

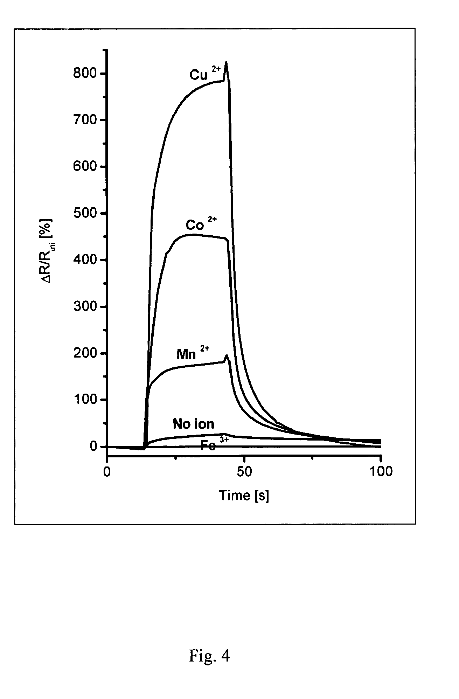

For building up a sensor array 5 sensors were prepared in one run (simultaneously) using nanoparticle coatings of the same composition (compare example 1) and 4 of them immersed in isopropanol solutions of different metal ions. One sensor was treated with pure isopropanol as a reference. Figure 4 shows the sensor responses towards 3030 ppm 1-butylamine. It can be clearly seen that the sensor response varies strongly for the sensors treated with different metal ions. The highest response was observed for Cu doped films, whrereas the response of the Fe doped film is even less that those of the undoped material.

### Example 5:

### Cadaverine detection with AuDT doped with different metals

For building up a sensor array 4 AuDT sensor coatings were assembled (compare example 2) and immersed in different metal ion solutions in methanol. Figure 5 shows the sensor responses towards 20ppb cadaverine. It can be clearly seen that the sensor response varies strongly for the sensors treated with different metal ions. The highest response was observed for Cu doped films.

### Example 6:

### Methylmercaptane detection with AuDT doped with different metals

For building up a sensor array 9 AuDT sensor coatings were assembled (compare example 2) and immersed in different metal ion solutions in methanol. Figure 6 shows the sensor responses towards 20 ppb methylmercaptane. It can be clearly seen that the sensor response varies strongly for the sensors treated with different metal ions. The highest responses were observed for Ag and Hg doped films.

The features of the present invention disclosed in the specification, the claims and/or in the accompanying drawings, may, both separately, and in any combination thereof, be material for realising the invention in various forms thereof.

## Claims

1. Use of a nanoparticle film said nanoparticle film having metal ions incorporated, for detecting the presence or absence of a gaseous or volatile or liquid analyte.

2. Use according to claim 1, wherein, in said film, nanoparticles are linked by organic linkers.

3. Use according to any of claims 1-2, wherein said gaseous or volatile or liquid analyte is a metabolite of an organism.

4. Use according to any of claims 1-3, wherein said gaseous or volatile or liquid analyte is a nitrogen containing compound or a sulphur containing compound.

5. Use according to any of claims 1-4, wherein said gaseous or volatile or liquid analyte is selected from the group comprising volatile sulphur compounds, such as methylmercaptane, hydrogensulfide, dimethylsulfide, biological amines, such as cadaverine, 1-butylamine, methylamine, isobutylamine, putrescine, histamine, tyramine, indole, skatole and phenethylamine.

6. Use according to any of claims 1-5, wherein said metal ions are selected from the group comprising Mg²⁺, Ca²⁺, Pb^{2+/4+}, Mn^{2+/3+/4+/6+/7+}, Co^{2+/3+}, Fe^{2+/3+}, Cu^{+/2+}, Ag⁺, Zn²⁺, Cd²⁺ Hg^{+/2+}, Cr^{2+/3+/6+}, Ce^{3+/4+}, Pd^{2+/4+}, Pt^{2+/4+6+}, Cu^{+/2+}, Fe²⁺/³⁺, Sn⁴⁺, Au^{+/2+/3+/5+}, Ni ²⁺, Rh^{+/2+/3+/4+}, Ru^{2+/3+/4+/6+/8+}, MO^{2+/3+/4+/5+/6+}

7. Use according to any of claims 1-6, wherein said metal ions have been incorporated in said nanoparticle film by exposing said nanoparticle film, to a solution of metal ions, said film, prior to said incorporations of metal ions, being devoid of metal ions.

8. Use according to any of claims 1-7, wherein said metal ions incorporated in said film are not complexed in coordination complexes before incorporation and are not coordinated by carboxylate groups of mercaptoundecanoic acid.

9. Use according to any of the foregoing claims, wherein said metal ions, upon incorporation into said film, react with said film and become oxidised or reduced, preferably to a metallic state.

10. Use according to any of claims 1-9, wherein said gaseous or volatile or liquid analyte is present in a medium to which said nanoparticle film is exposed, and said analyte is present in said medium at a concentration in the range of from 10 parts per trillion to 100 parts per million.

11. Use according to any of the foregoing claims, wherein said analyte is an amine and said metal ions are Cu²⁺.

12. Use according to any of claims 1-10, wherein said analyte is methylmercaptane and said metal ions are Ag⁺ or Hg²⁺ or combinations thereof.

13. Use according to any of claims 1-12, wherein said organic linkers are bi- or polyfunctional linkers, preferably selected from the group comprising thiols such as C₅-C₃₀-alkane dithiols, such as 1,12-dodecanedithiol, or amines or dithiocarbamates such as 1,4,10,13-tetraoxa-7,16-bisdithiocarbamate-cyclo-octadecane of thiocticacids or isocyanates.

14. A method of detecting a gaseous or volatile or liquid analyte in a medium, said method comprising:
providing a sensor comprising a nanoparticle film, in which film, preferably, said nanoparticles are linked by organic linkers, said film having metal ions incorporated, said nanoparticle film showing a change in electrical conductivity in the presence of a gaseous or volatile or liquid analyte,
exposing said sensor to said medium, and measuring a response based on said change of conductivity if said analyte is present, said film, said metal ions and said analyte being as defined in any of claims 1-13.
